# EUROPÄISCHE PATENTSCHRIFT

(11) **EP 2 147 646 B1**
(45) Veröffentlichungstag und Bekanntmachung des Hinweises auf die Patenterteilung: **28.01.2015**
(21) Anmeldenummer: 09166106.6
(22) Anmeldetag: 22.07.2009
(51) Int. Cl.: A61B 17/34

(54) **Medizinisches Instrument mit einem flexiblen Dichtungssystem**
Medical instrument with a flexible seal system
Instrument médical doté d'un système d'étanchéité flexible

(30) Priorität: 23.07.2008 DE 102008035310
(43) Veröffentlichungstag der Anmeldung: 27.01.2010
(73) Patentinhaber: Karl Storz GmbH & Co. KG, 78532 Tuttlingen (DE)
(72) Erfinder: Teichtmann, Elmar, 75031 Eppingen (DE); Sauer, Michael, 78532, Tuttlingen (DE)
(74) Vertreter: Witte, Weller & Partner Patentanwälte mbB

(56) Entgegenhaltungen:
- EP-A1- 0 696 459
- WO-A1-2007/121425
- US-A- 5 628 732

## Beschreibung

Die vorliegende Erfindung betrifft ein medizinisches Instrument, mit einer Kanüle, durch die Schäfte weiterer Instrumente mit unterschiedlichen Schaftdurchmessern hindurchführbar sind, mit einer Dichtung, die eine Öffnung mit veränderbarem Öffnungsquerschnitt aufweist, durch den die Schäfte mit unterschiedlichen Schaftdurchmessern dicht hindurchführbar sind, mit einer Spreizvorrichtung zum Spreizen der Öffnung der Dichtung, wobei die Spreizvorrichtung einen Spreizkonus aufweist, der mehrere Lamellen aufweist, die schwenkbar an einem Ringkörper angebracht sind und einen sich von proximal nach distal verjüngenden Spreizkörper bilden, der mit der Dichtung im Bereich der Öffnung in Verbindung steht.

Ein solches medizinisches Instrument ist aus der EP 0 696 459 B1 bekannt.

Bei diesem medizinischen Instrument handelt es sich um einen Trokar, der in der minimal-invasiven Chirurgie eingesetzt wird. Dabei wird der entsprechende Trokar durch eine kleine Öffnung, die z.B. durch einen kleinen Schnitt herbeigeführt werden kann, mittels eines Trokardorns in den Körper des Patienten eingebracht. Nach Entfernen des Trokardorns werden durch diesen Trokar dann Schäfte von anderen Instrumenten, die für den chirurgischen Eingriff verwendet werden, eingeführt. Zu diesen Instrumenten zählen z.B. Greifwerkzeuge, Koagulationsinstrumente, Endoskope und dergleichen. Diese weisen unterschiedliche Durchmesser auf.

Da solche minimal-invasiven Eingriffe häufig unter Insufflation des Innenraums, in dem die Operation stattfindet, durchgeführt werden, müssen alle Eingriffsstellen gasdicht sein.

Bei der eingangs erwähnten EP 0 696 459 B1 ist dazu eine elastische kegelförmige Dichtung in dem Trokar angeordnet, die sich von proximal nach distal verjüngt. Um eine gleichmäßige Aufweitung der Dichtung zu ermöglichen, wenn ein Instrument in den Trokar eingeführt wird, ist proximal von dieser Dichtung der Spreizkonus angeordnet. Ein in den Spreizkonus eingeführtes Instrument spreizt diesen radial und dieser weitet die Öffnung in der Dichtung auf. Neben der einfacheren Aufweitung der Dichtung verhindert der Spreizkonus ferner die Beschädigung der Dichtung beim Einführen von scharfkantigen Instrumenten, weil er aus einem harten Material hergestellt werden kann. Auf diese Weise wird die Dichtung vom Spreizkonus geschützt.

Zum Aufbau des Spreizkonus sind die Lamellen schwenkbar an einem Ring angebracht, wodurch sich der konische Körper des Spreizkonus ergibt und die Lamellen radial verschwenkt werden können. Dazu weisen diese an einem Ende Achsstifte auf, mit denen die Lamellen schwenkbar in entsprechende Aussparungen im Ringkörper eingehängt werden müssen.

Zu diesem Zweck benötigt somit jede Lamelle exakt geformte Abschnitte mit Achsstiften. Ferner muss der Ringkörper entsprechend exakt geformte Einhängevorrichtungen aufweisen, wobei zusätzlich noch eine passgenau geformte Abdeckung auf die Vorrichtung gesetzt werden muss, damit die Lamellen nicht vom Ring abfallen. Die Konstruktion erfordert also mehrere exakt gefertigte Einzelteile mit relativ aufwendigen Details. Dies ist besonders aufgrund der geringen Größe der Bauelemente, die im Millimeterbereich liegt, schwierig und macht daher die Konstruktion aufwendig. Gleiches gilt auch für die Montage, bei der die sehr kleinen Achsstifte in die entsprechenden Aussparungen im Ringkörper eingehängt werden müssen.

Es ist daher Aufgabe der vorliegenden Erfindung, ein medizinisches Instrument dieser Art dahingehend weiterzuentwickeln, dass das Dichtungssystem einfacher aufgebaut, weniger aufwendig zu montieren und kostengünstiger herzustellen ist.

Erfindungsgemäß wird die Aufgabe zum einen dadurch gelöst, dass die Lamellen über Filmscharniere mit dem Ringkörper verbunden sind.

Die bewegbare Verbindung zwischen Lamellen und Ringkörper mittels Filmscharnieren ermöglicht die Fertigung des Spreizkonus als ein einziges Werkstück durch ein Spritzgussverfahren. Dadurch wird der notwendige Aufwand zum Herstellen des Spreizkonus stark reduziert, da nur ein einziges Teil produziert werden muss und kein Zusammenbau von Einzelteilen nötig ist. Dies spart Zeit und Kosten.

Erfindungsgemäß wird die Aufgabe zum anderen dadurch gelöst, dass die Lamellen Kugelköpfe aufweisen, über die diese in entsprechende Öffnungen im Ringkörper einhängbar sind.

Bei dieser Lösung besteht der Spreizkonus zwar aus mehreren Teilen, das Vorsehen von Kugelköpfen, die in die entsprechenden Öffnungen des Ringkörpers eingelegt werden müssen, stellt aber eine einfache konstruktive Maßnahme dar. Die Kugelköpfe sind ohne besondere Aufmerksamkeit in die Öffnungen des Ringkörpers einzulegen, wodurch die Montage einfach ist.

In einer Ausgestaltung werden die Kugelköpfe der Lamellen durch einen Abdeckring im Ringkörper gehalten.

Die Verwendung eines Abdeckrings verhindert das ungewollte Herabfallen der Kugelgelenk-gelagerten Lamellen aus dem Ringkörper. Sie unterstützt ferner das Konzept der einfachen Konstruktion, da der Abdeckring für eine sichere Halterung der Kugelgelenk-gelagerten Lamellen sorgt.

In einer weiteren Ausgestaltung erstreckt sich die Dichtung vom distalen Ende des Spreizkonus nach distal weg.

Die Wahl dieser konstruktiven Anordnung hat den Vorteil, dass zum einen das Zusammenfügen von Spreizkonus und Dichtung einfach vonstatten geht, da beide Bauteile über gut zugängliche Außenseiten miteinander verbunden werden. Zum anderen unterstützt die dabei vorliegende Anordnung der Dichtung die Entnahme von Gewebeproben, da sie auf diese Weise eine trichterartige Form darstellt, die von distal nach proximal verjüngt. Wird z.B. mit einem Greifwerkzeug eine solche Probe genommen und anschließend durch die Trokarhülse nach proximal geführt, so gelangt diese schließlich an das Dichtungssystem. Dort wird sie aufgrund des allmählich geringer werdenden Durchmessers der Dichtung zur proximalen schmalen Öffnung der Dichtung geführt und ggf. auch in der Form angepasst. Ein ungewolltes Hängenbleiben aufgrund einer abrupten Durchmesseränderung im Verlauf des Herausziehens der Probe wird somit vermieden.

In einer weiteren Ausgestaltung der Erfindung umgibt die Dichtung den Spreizkonus.

Diese Ausgestaltungsform führt zu einem kompakten Dichtungssystem. Aufgrund der geringen Größe ist dieses kompatibel mit vielen bekannten Trokarsystemen. Da es aufgrund dieses kompakten Aufbaus vormontiert und gut gehandhabt werden kann, kann es außerdem einfach in die Trokarsysteme eingesetzt werden.

In einer weiteren Ausgestaltung der Erfindung ist das distale Ende der Lamellen des Spreizkonus formschlüssig mit einer Fassung am Rand der Öffnung in der Dichtung verbunden.

Durch diese Verbindung des distalen Endes der Lamellen mit der Fassung am Rand der Öffnung der Dichtung wird ein Verrutschen der Lamellen beim Spreizen der Dichtung verhindert. Somit wird einem möglichen Lösen der Lamellen vom Rand entgegengewirkt und ein gleichmäßiges Spreizen sichergestellt. Ferner führt die Verbindung dazu, dass sich die Lamellen aufgrund der Rückstellkraft der gespreizten elastischen Dichtung nach dem Entfernen eines Schaftes aus dem Trokar wieder zusammen mit der Dichtungsöffnung zurück in die Ausgangsposition bewegen.

In einer Ausgestaltung der Erfindung ist das distale Ende der Lamellen des Spreizkonus mit der Dichtung verklebt.

Ein Verkleben zwischen Lamellen und Öffnung der Dichtung sorgt für einen unlösbaren festen Kontakt zwischen den Bauteilen. Hierdurch wird ein ungewolltes Trennen der Bauteile verhindert, so dass die Lamellen stets an der Öffnung der Dichtung angeordnet sind. Das Verkleben ist als Einzelmaßnahme, aber auch in Kombination mit der formschlüssigen Verbindung denkbar.

In einer weiteren Ausgestaltung der Erfindung ist die Dichtung zusammen mit dem Spreizkonus in einem Hüllkörper angeordnet.

Die Verwendung eines Hüllkörpers gibt dem System aus Spreizkonus und Dichtung eine erhöhte Stabilität. Der Zusammenbau aus dem Spreizkonus aus steifem Material und der Dichtung aus elastischem Material stellt ein in seitlicher Richtung relativ instabiles Gebilde dar. Dies ist insbesondere bei der Ausgestaltung ausgeprägt, bei der sich Spreizkonus und Dichtung ausgehend von der Verbindungsstelle untereinander diametral voneinander weg erstrecken. Ein seitlich versetzt oder schräg in den Spreizkonus eingeführtes Instrument würde ein seitliches Ausweichen oder einen seitlichen Versatz des Zusammenbaus bewirken. Der Hüllkörper wirkt dem entgegen, lässt aber eine gewisse Flexibilität zu.

In einer weiteren Ausgestaltung der Erfindung besteht der Hüllkörper aus einem Doppelkugelgelenk, das vorzugsweise aus einer Dichtungshalterung, einem Kugelabschnitte aufweisenden Mittelteil und einer proximalen Kugelfassung besteht.

Die Ausgestaltung des Hüllkörpers in Form eines in gewissem Maße beweglichen Doppelkugelgelenks gestattet es dem Spreizkonus und der mit diesem verbundenen Dichtung, gewisse Kipp- oder Versatzbewegungen durchzuführen. Dies ist besonders bei Instrumenten, deren Schäfte einen kleinen Durchmesser aufweisen, sinnvoll. Bei Instrumenten mit dünnen Schäften kann es zu einem Versatz von dem mittigen Durchgang durch die Öffnung der Dichtung kommen. Ferner kann es insbesondere bei dünnen biegbaren Schäften zu einem krummen Verlauf des Schaftes im Trokar kommen. Beides führt dazu, dass die Dichtung nicht gleichmäßig auf dem ganzen Umfang des Schaftes mit diesem in Kontakt kommt. Die Folge wären unerwünschte Undichtigkeiten.

Aufgrund des Hüllkörpers in Form eines Doppelkugelgelenks kann sich das Dichtungssystem so verschieben, dass der Schaft wieder mittig durch die Öffnung der Dichtung verläuft. Dadurch wird die gewünschte Dichtigkeit des Trokarsystems erhalten.

In einer weiteren Ausgestaltung der Erfindung besteht der Hüllkörper aus einem flexiblen Hüllrohr.

Die Verwendung eines flexiblen Hüllrohrs ermöglicht ebenfalls eine sehr gute Anpassung der Dichtung an den Versatz eines eingeführten Instruments, da solch ein flexibles Hüllrohr ebenfalls eine Beweglichkeit des Dichtungssystems ermöglicht. Durch das Anpassen der Dichtung an einen Versatz oder ein schräges Durchlaufen des Schaftes durch die Öffnung der Dichtung wird folglich auch die Dichtigkeit des Trokarsystems sichergestellt.

In einer weiteren Ausgestaltung der Erfindung besteht das flexible Hüllrohr aus einer im Wesentlichen parallelen Aneinanderreihung von Ringelementen, wobei vorzugsweise zwei benachbarte Ringelemente an zwei sich radial gegenüberliegenden Stellen miteinander verbunden sind und weiterhin die Verbindungsstellen eines Ringelements zu dem distal nächsten Ringelement jeweils um 90° gegenüber den Verbindungsstellen zu dem proximal nächsten Ringelement kreisförmig versetzt angeordnet sind.

Die abwechselnd um 90° kreisförmig versetzt angeordneten Verbindungsstellen zwischen den Ringelementen erlauben eine Flexibilität des Hüllrohrs in alle seitlichen Richtungen, da aufgrund der radial gegenüberliegenden Anordnung der Verbindungsstellen die zusammengehörigen Ringelemente eine Kippachse aufweisen. Durch diese können sie aus der zueinander parallelen Anordnung an umfänglich zwischen den Verbindungsstellen liegenden Stellen aufeinander zu gekippt werden. Der Versatz um 90° von einem zum nächsten Ringelementpaar sorgt in der Gesamtanordnung der Ringelemente dafür, dass ein Abkippen in zwei senkrecht zueinander verlaufende Richtungen möglich ist. Das heißt, der so erzeugte Hüllkörper kann an seinem freien proximalen Ende in zwei voneinander unabhängige Richtungen und somit in der durch diese Richtungen gegebenen Ebene bewegt werden. Neben der guten Beweglichkeit und der damit verbundenen Fähigkeit, einen Versatz eines Schaftes im Trokar auszugleichen, wird durch die um 90° zueinander versetzt angeordneten Verbindungsstellen gleichzeitig eine gute axiale Stabilität erreicht, da im Belastungsfall eine Gleichverteilung der einwirkenden Kräfte vorliegt.

Ein weiterer Vorteil bei der Verwendung von Ringelementen liegt darin, dass diese wenig anspruchsvoll zu fertigende Bauteile sind, was die Herstellung einfach macht.

In einer Ausgestaltung der Erfindung sind die Verbindungen flexible Stege.

Der Vorteil dieser Ausgestaltung besteht darin, dass die schmalen und damit flexiblen Stege direkt ohne mehrteilige Gelenke an die jeweiligen Elemente angeordnet sind. Dadurch kann das so aufgebaute flexible Hüllrohr als ein einziges Werkstück z.B. aus Kunststoff durch ein Spritzgussverfahren hergestellt werden. Da hierdurch ein nachträgliches Montieren der Ringelemente entfällt, erfolgt die Herstellung sehr schnell und einfach.

In einer Ausgestaltung der Erfindung sind die Verbindungen Kippgelenke.

Durch die Konstruktion des flexiblen Hüllrohrs aus einzelnen Ringelementen und durch deren Verbindung mittels Kippgelenken wird die Länge des flexiblen Hüllrohrs variabel. Sie kann somit bei der Konstruktion eines Dichtungssystems an die Länge des Spreizkonus und an die Länge der Dichtung angepasst werden. Mit einem Ringelementtyp können somit unterschiedlich lange Hüllrohre aufgebaut werden, was diese Ausgestaltungsform sehr variabel macht.

In einer Ausgestaltung der Erfindung sind die Ringelemente an den Stellen einer Kippgelenkfassung höher als an denen eines Kippgelenkeinsatzes.

Die Stelle der Kippgelenkfassung im Ringelement muss, um den kompletten Kippgelenkeinsatz aufnehmen zu können, eine gewisse Höhe aufweisen. Diese Höhe ist an den übrigen Stellen des Ringelements geringer. Dadurch wird der Bereich, in dem die Ringelemente zueinander verkippt werden können, vergrößert, da der Abstand zwischen den Ringelementen an den umfänglich zwischen den Kippgelenken liegenden Stellen größer ist. Auf diese Weise wird ein größtmöglicher Neigungswinkel erreicht, was die Flexibilität des Hüllrohrs erhöht. Dadurch kann sich das Dichtungssystem besser an jeglichen Versatz eines Schaftes in der Öffnung der Dichtung anpassen.

In einer weiteren Ausgestaltung der Erfindung weitet sich das flexible Hüllrohr von proximal nach distal konisch auf.

Durch diese Aufweitung wird der Hüllkörper an den Durchschnittsunterschied zwischen dem Ringkörper des Spreizkonus und dem zumeist durchschnittsgrößeren distalen Ende der Dichtung angepasst. Dadurch wird einerseits ein guter Halt am distalen Ende der Dichtung und dem Trokargehäuse, an dem diese angeordnet ist, und andererseits am Spreizkonus sichergestellt. Dies führt zu der gewünschten Stabilität.

In einer weiteren Ausgestaltung der Erfindung besteht der Spreizkonus aus acht bis zwölf, vorzugsweise aus zehn Lamellen.

Die Verwendung von acht bis zwölf bzw. bevorzugt zehn Lamellen ermöglicht einerseits ein gleichmäßiges Aufweiten der Öffnung der Dichtung, da die Anzahl der verwendeten Lamellen so groß ist, dass die beim Aufweiten entstehenden Zwischenräume möglichst klein sind und außerdem der Abstand zwischen zwei Lamellen im gespreizten Zustand klein ist. Somit wird auch im gespreizten Zustand durch die Lamellen immer noch eine gleichmäßige runde Form an der Öffnung der Dichtung gebildet. Dadurch wird insbesondere die Dichtigkeit an der Öffnung der Dichtung während und nach dem Einführen eines Schaftes begünstigt. Andererseits weisen diese Lamellen aber auch eine genügend große Stabilität auf, da sie so noch eine ausreichend große Breite besitzen. Somit werden sie nicht beim Einführen speziell scharfkantiger Instrumente beschädigt oder zerstört, wodurch weiterhin der Schutz der Dichtung gegeben ist.

In einer weiteren Ausgestaltung der Erfindung verjüngen sich die einzelnen Lamellen von proximal nach distal.

Gerade im ungespreizten Zustand des Spreizkonus sollte dieser eine möglichst geschlossene Fläche aufweisen, um beim Einführen eines Schaftes keine Freiräume zu bieten, durch die der Schaft an die Dichtung gelangen kann und diese somit eventuell beschädigt. Da ein kegelförmiger Körper vorliegt, ist der Umfang am Ringkörper deutlich größer als auf Höhe der Öffnung der Dichtung. Im Falle von Lamellen, die im Verlauf konstant die gleiche Breite aufweisen und bei denen schon am Ringkörper eine geschlossene Fläche vorliegen soll, führt dies am distalen Ende des Spreizkonus dazu, dass sich die Lamellen überlappen. Dies kann wiederum dazu führen, dass sich die Lamellen verkanten und somit die Funktionsweise des Spreizkonus behindern.

Aufgrund der Verjüngung der Lamellen kann es hingegen im ungespreizten Zustand nicht zu unerwünschten Überlappungen oder Verkantungen der einzelnen Lamellen untereinander kommen. Dies garantiert die unbeeinträchtigte Funktionsweise des Spreizkonus und bei kontinuierlich abnehmender Breite trotzdem noch einen ungespreizten Spreizkonus mit nahezu geschlossener Innenfläche.

In weiteren Ausgestaltungen der Erfindung sind die Lamellen, der Ringkörper und der Hüllkörper aus Hartplastik gefertigt.

Die Verwendung von Hartplastik für die Lamellen, den Ringkörper und den Hüllkörper stellt im vorliegenden Fall einen guten Kompromiss dar, da einerseits eine ausreichende Stabilität z.B. beim Einführen von scharfkantigen Instrumenten erhalten wird und trotzdem eine einfache Herstellung, beispielsweise durch das Spritzgussverfahren, ermöglicht wird.

In einer weiteren Ausgestaltung der Erfindung ist die Dichtung aus einem elastischen Material gefertigt.

Die Verwendung eines elastischen Materials für die Dichtung ermöglicht einerseits ein geeignetes Aufweiten der Öffnung der Dichtung durch den Spreizkonus beim Einführen des Schaftes eines Instruments und andererseits eine gleichzeitig optimale Abdichtung am Schaft eines solchen Instruments aufgrund des dichten Kontakts zwischen elastischem Material und Schaft. Dieser wird insbesondere dadurch erreicht, dass die gespreizte Öffnung der Dichtung aufgrund der Rückstellkraft des elastischen Materials gegen den eingeführten Schaft umlaufend gepresst wird.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert. Es zeigen:
- Fig. 1: eine perspektivische Seitenansicht eines medizinischen Instruments mit einem flexiblen Dichtungssystem,
- Fig. 2: einen Schnitt längs der Linie II-II aus Fig. 1,
- Fig. 3: ein Dichtungssystem des medizinischen Instruments von proximal betrachtet,
- Fig. 4: eine perspektivische Darstellung eines flexiblen Hüllrohrs des Dichtungssystems,
- Fig. 5: einen Schnitt längs der Linie V-V aus Fig. 3,
- Fig. 6: eine perspektivische Darstellung eines Spreizkonus,
- Fig. 7: eine perspektivische Darstellung einer Dichtung,
- Fig. 8: eine abschnittsweise Schnittansicht eines Dichtungssystems, wobei die Dichtung den Spreizkonus umgibt,
- Fig. 9: eine perspektivische Darstellung eines flexiblen Hüllrohrs mit Kippgelenken,
- Fig. 10: eine Schnittansicht eines Dichtungssystems mit einer Doppelkugellagerung als einen Hüllkörper,
- Fig. 10a: ein Dichtungssystem wie in Fig. 10, jedoch mit ausgelenktem Hüllkörper,
- Fig. 11: eine perspektivische Explosionsansicht der Doppelkugellagerung aus Fig. 10,
- Fig. 12: eine perspektivische Ansicht eines Spreizkonus mit Kugelkopfaufweisenden Lamellen,
- Fig. 13: die Kugelkopf-aufweisende Lamelle in perspektivischer Einzelansicht,
- Fig. 14: eine Schnittansicht des Dichtungssystems mit Doppelkugellagerung mit einem angesetzten Schaft,
- Fig. 14a: eine Schnittansicht des Dichtungssystems mit Doppelkugellagerung, wobei der Schaft eingeführt ist.

Ein in den Figuren dargestelltes medizinisches Instrument ist in seiner Gesamtheit mit der Bezugsziffer 10 bezeichnet.

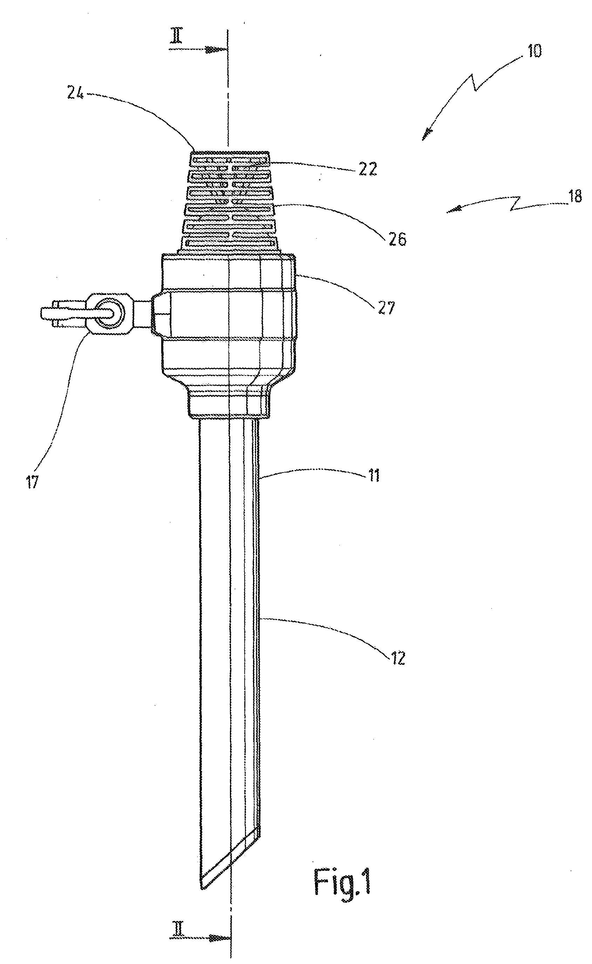

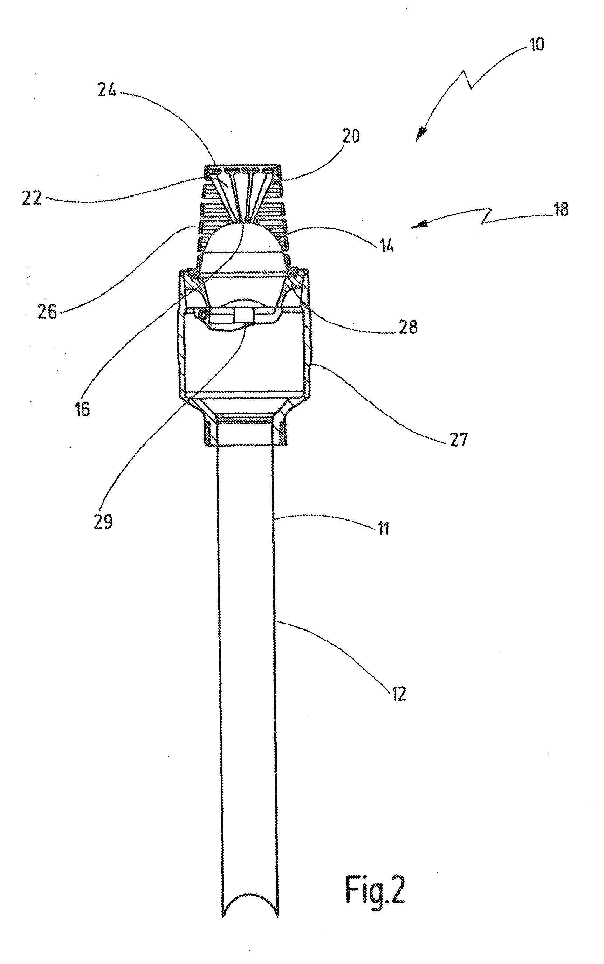

Das dargestellte medizinische Instrument 10 ist ein Trokar und weist distal eine Trokarhülse 11, die hier durch eine Kanüle 12 gebildet wird, mittig ein Trokargehäuse 27 und proximal an diesem angeordnet ein Dichtungssystem 18 auf. Fig. 1 zeigt ferner ein am Trokargehäuse 28 angeordnetes Ventil 17, welches z.B. als Zuleitung für Gase dienen kann. Diese werden dazu verwendet, eine Insufflation der Operationsstelle durchzuführen, um dadurch einen besseren Zugang zu den Organen, Gefäßen, Geweben oder dergleichen, an denen die Operation durchgeführt werden soll, zu erhalten. Um ein ungewolltes Austreten der Gase durch den Trokar in proximale Richtung zu verhindern, ist distal des Dichtungssystems 18 ein selbstschließender Verschluss 29 angeordnet (Fig. 2). Dieser verschließt die proximale Öffnung, sobald kein Schaft in das medizinische Instrument 10 eingeführt ist.

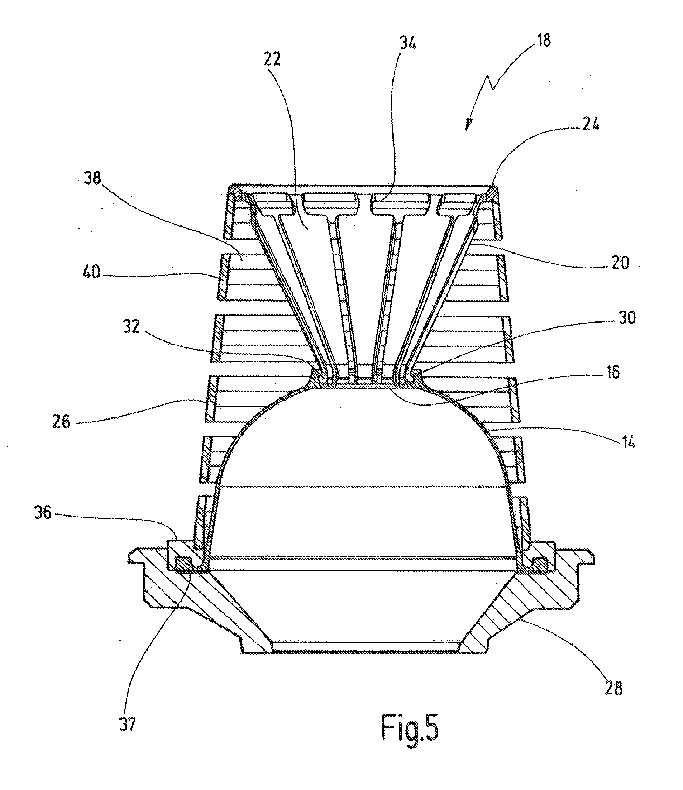

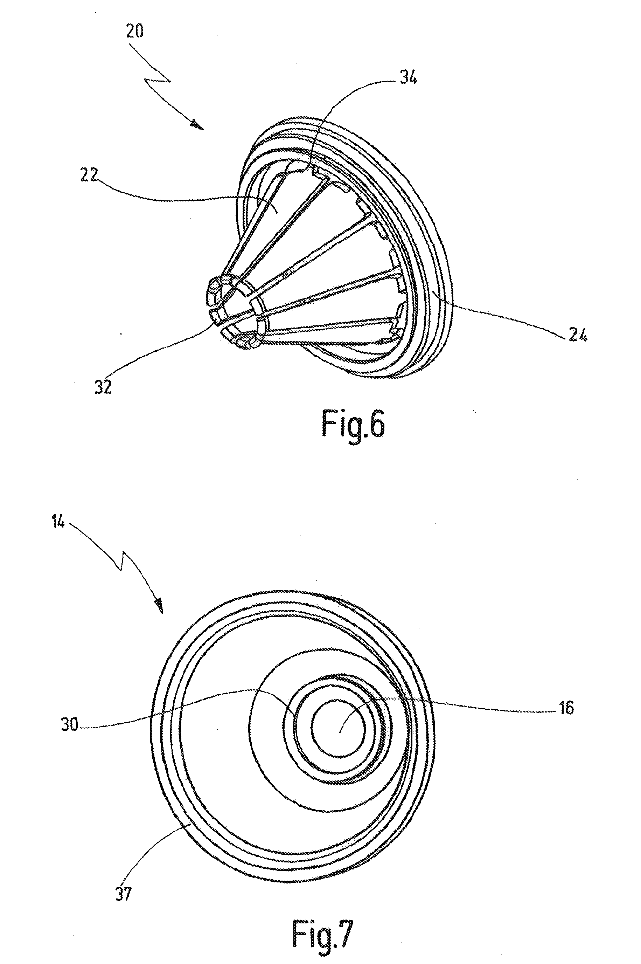

Wie in den Fig. 1, 2 und 5 zu sehen ist, weist das Dichtungssystem 18 eine Dichtung 14 auf. Diese ist mit einem Dichtungsrand 37 durch einen Befestigungsring 36 an einer Dichtungshalterung 28 befestigt, mit der das Dichtungssystem 18 am Trokargehäuse 27 angebracht ist. Sie erstreckt sich proximal von der Dichtungshalterung 28 weg und verjüngt dabei. An ihrem proximalen Ende weist die Dichtung 14 eine Öffnung 16 sowie eine diese Öffnung 16 umgebende Fassung 30 auf (Fig. 5 und 7). An der Fassung 30 befestigt und sich von der Dichtung 14 proximal weg erstreckend ist ein Spreizkonus 20 angeordnet (Fig. 5).

Wie in Fig. 5 und 6 zu sehen ist, ist dieser Spreizkonus 20 aus einem Ringkörper 24, an dem Lamellen 22 mittels Folienscharnieren 34 verschwenkbar ringförmig distal ausgerichtet angeordnet sind, aufgebaut. Diese Lamellen 22 weisen am distalen Ende Abschlüsse 32 auf, die formschlüssig mit der Fassung 30 der Dichtung 14 verbunden werden können. Durch den Querschnittsunterschied zwischen dem Ringkörper 24 und der Fassung 30 erhält der Spreizkonus 20 seine konische Form. Die Lamellen 22 verjüngen von proximal nach distal in der Art, dass sich eine gleichmäßige nahezu geschlossene innere Fläche des Spreizkonus 20 im ungespreizten Zustand ergibt.

Durch die Verbindung zwischen den Lamellen 22 und der Dichtung 14, wobei die Abschlüsse 32 in der Fassung 30 eingelegt sind, werden bei Einführen eines Schaftes durch diesen zunächst die Lamellen 22 radial nach außen gedrückt, wodurch gleichzeitig die Öffnung 16 der Dichtung 14 gespreizt wird, ohne jedoch selber direkt durch den Schaft gespreizt zu werden.

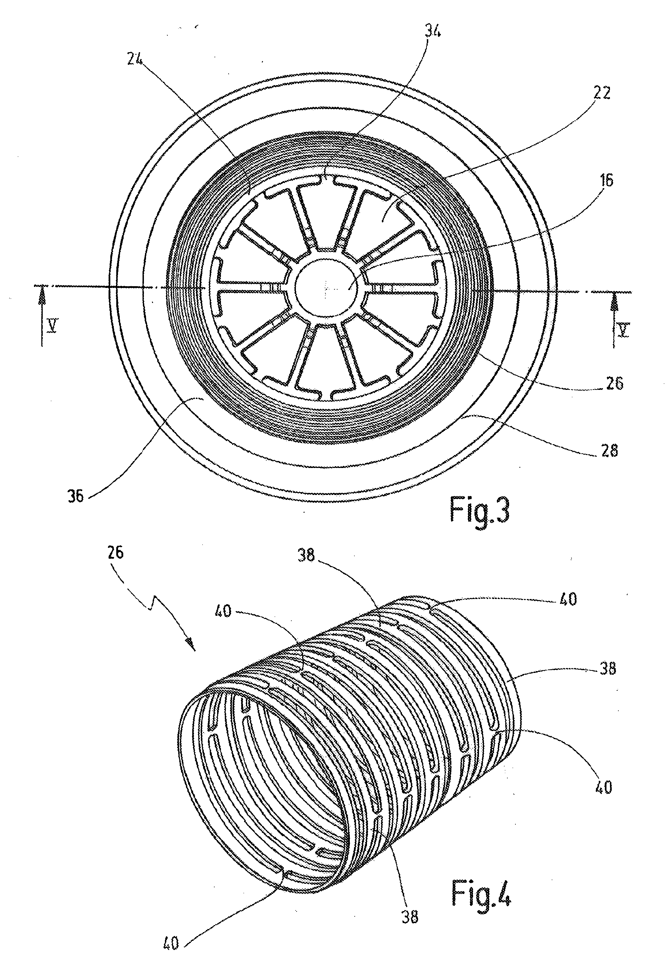

Das Gebilde aus der Dichtung 14 und dem Spreizkonus 20 wird von einem Hüllrohr 26 umhüllt. Dazu ist dieses Hüllrohr 26 distal mit der Dichtungshalterung 28 und proximal mit dem Ringkörper 24 verbunden (Fig. 5). Das Hüllrohr 26 besteht aus einzelnen Ringelementen 38, die über jeweils radial gegenüberliegende Stege 40 miteinander verbunden sind (Fig. 4). Um eine Flexibilität des Hüllrohrs zu erhalten, wird dieses einerseits aus einem ausreichend elastischen Material gefertigt, so dass eine Flexibilität der Stege 40 gegeben ist und die Stege 40 außerdem von einem zum nächsten Ringelementpaar jeweils um 90° kreisförmig versetzt zueinander angeordnet.

Um das Hüllrohr 26 kann noch eine zusätzliche, hier nicht gezeigte Kappe angeordnet sein, die eine durchgehende äußere Oberfläche aufweist. Dadurch wird das durchlässige Hüllrohr 26 verschlossen, so dass keine Verunreinigungen in den Zwischenraum zwischen Hüllrohr 26 und Spreizkonus 20 und Dichtung 14 eindringen und sich festsetzen können.

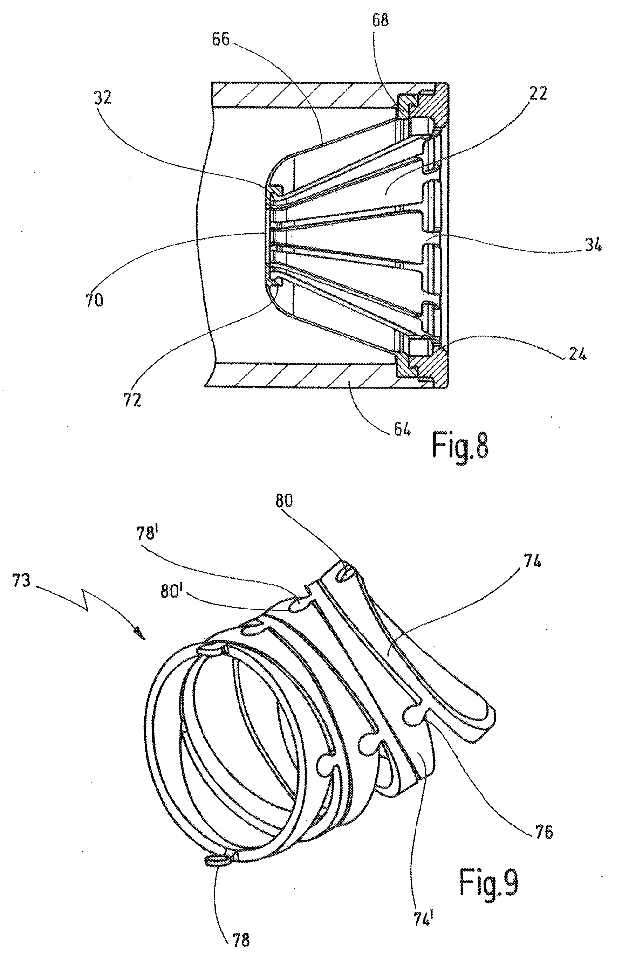

Im Gegensatz zu der bisher beschriebenen Variante, bei denen sich der Spreizkonus 20 proximal vom proximalen Ende der Dichtung 14 weg erstreckt, ist im Ausführungsbeispiel von Fig. 8 die Dichtung 66 so angeordnet, dass sie von proximal nach distal verjüngt und dabei den Spreizkonus 20 umgibt. Dazu ist sie mit einem Befestigungsring 68 an einem Trokargehäuse 64 angeordnet, an dem auch proximal der Ringkörper 24 des Spreizkonus 20 befestigt ist. Das distale Ende der Dichtung 66 bildet eine Öffnung 70, die von einer Fassung 72 umgeben ist. In dieser sind analog zum zuvor beschriebenen Ausführungsbeispiel die Abschlüsse 32 der Lamellen 22 formschlüssig angeordnet.

Ein weiteres Ausführungsbeispiel für ein flexibles Hüllrohr ist in Fig. 9 gezeigt. Im Gegensatz zu dem in Fig. 4 beschriebenen Hüllrohr 26, welches z.B. durch ein Spritzgussverfahren als ein einziges Teil hergestellt werden kann, zeigt Fig. 9 ein Hüllrohr 73, das aus separaten Ringelementen 74 aufgebaut ist. Diese Ringelemente 74 weisen zwei unterschiedliche Seiten auf. Während die eine Seite zwei Kippgelenkfassungen 80 aufweist, besitzt die andere Seite zwei Kippgelenkeinsätze 78. Die Kippgelenkeinsätze 78 und die Kippgelenkfassungen 80 sind für sich jeweils einander radial gegenüber angeordnet. Zueinander sind die Kippgelenkfassung 80 und der Kippgelenkeinsatz 78 an einem Ringelement 74 um 90° kreisförmig versetzt angeordnet.

Wie in Fig. 9 gezeigt, werden mehrere Ringelemente 74 durch Verbindung von den Kippgelenkeinsätzen 78 eines Rings mit den Kippgelenkfassungen 80 eines anderen Rings miteinander verbunden. Dadurch ergibt sich das Hüllrohr 73, welches mit seinen Verbindungen zwischen den Kippgelenkeinsätzen 78 und den Kippgelenkfassungen 80 den Stegen 40 zwischen den Ringelementen 38 des Hüllrohrs 26 ähnelt (Fig. 4 und Fig. 9). Der Kippgelenkeinsatz 78 und die Kippgelenkfassung 80 bilden so ein Kippgelenk 76, welches für die Flexibilität des Hüllrohrs 73 verantwortlich ist.

Um einen möglichst großen Beweglichkeitsbereich zu erhalten, ist dabei an den Kippgelenkfassungen 80 das Ringelement 74 höher als an den Stellen des Kippgelenkeinsatzes 78 (Fig. 9). Das Ringelement 74 kann somit weiter um die Achse, die sich durch die einander radial gegenüberliegenden Kippgelenke 76 bildet, verschwenkt werden. Ein erstes Ringelement 74 kann somit an der Stelle der Kippgelenkfassung 80 weiter zu einem zweiten Ringelement 74' verschwenkt werden, als wenn letzteres an der Stelle des Kippgelenkeinsatzes 78' die gleiche Höhe aufweisen würde wie an der Stelle des Kippgelenks 76.

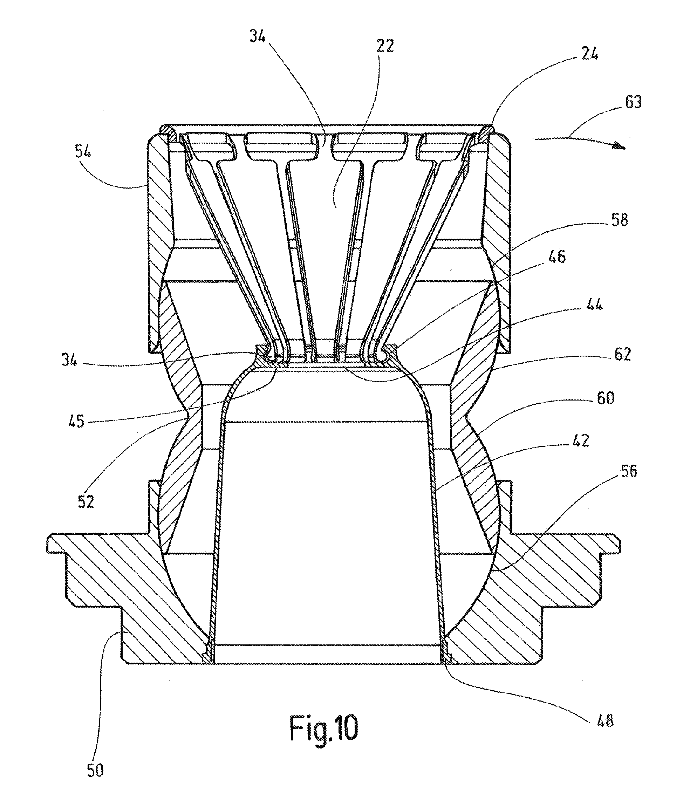

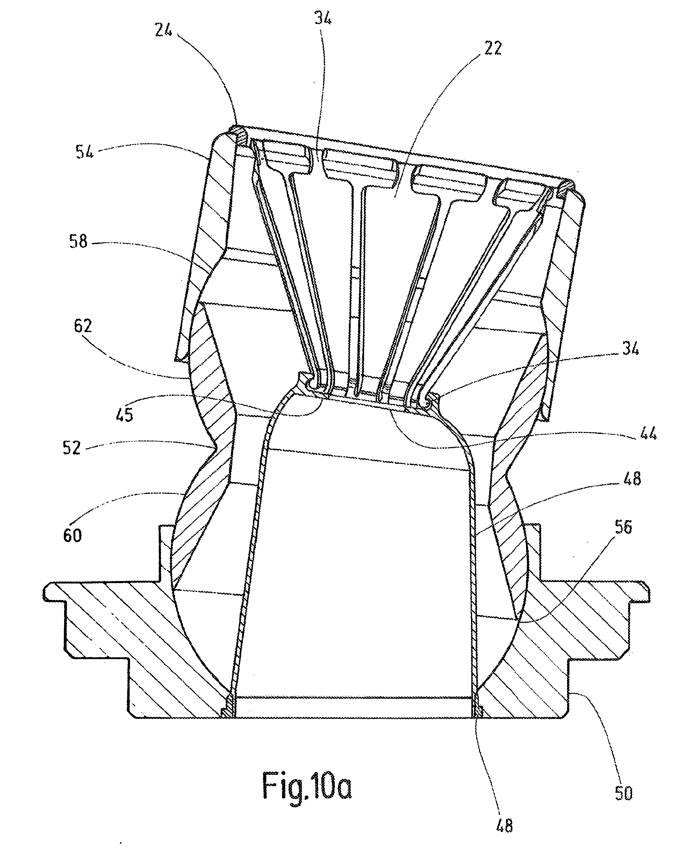

Ein weiteres Ausführungsbeispiel eines Hüllkörpers ist in Fig. 10 gezeigt.

Dieser Hüllkörper ist ein Doppelkugelgelenk 41 und wird aus einer Dichtungshalterung 50, einem Mitteilteil 52 und einer Kugelfassung 54 gebildet. Die Dichtungshalterung 50 weist eine kugelförmige Ausbuchtung 56 auf, in die das Mittelteil 52 eingesetzt wird. Das Mittelteil 52 seinerseits weist dafür zwei Kugeleinsätze 60 und 62 auf.

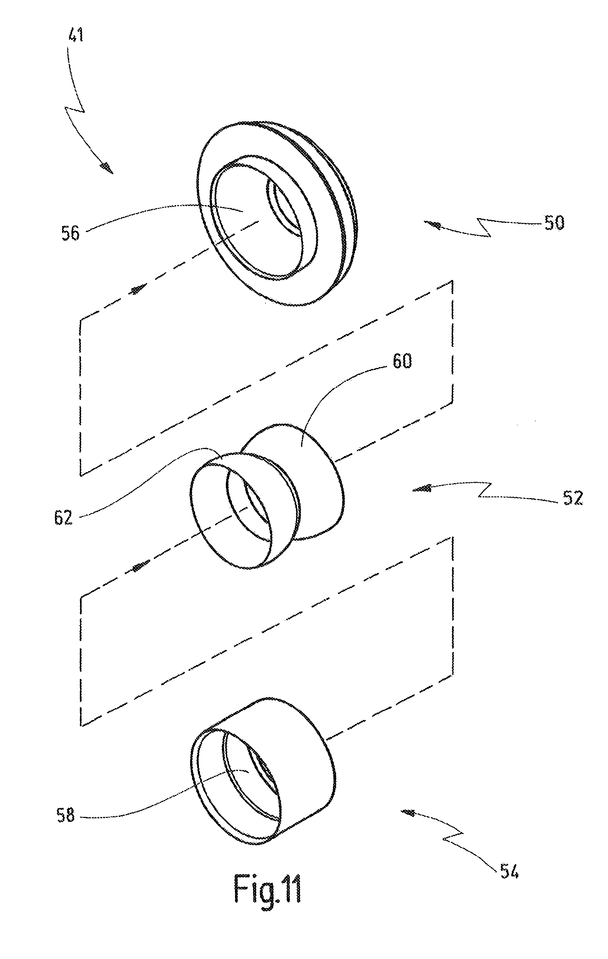

Der Kugeleinsatz 60 ist dabei in der kugelförmigen Ausbuchtung 56 angeordnet und in dieser beweglich. Proximal auf dem Kugeleinsatz 62 ist die Kugelfassung 54 mit einer kugelförmigen Ausbuchtung 58 beweglich angeordnet (Fig. 10 und 11).

Diese Vorrichtung bildet den Hüllkörper für eine Anordnung von Spreizkonus 20 und einer Dichtung 42 vergleichbar mit dem Dichtungssystem 18 von Fig. 5. Dabei ist die Dichtung 42 mit einem Dichtungsrand 48 an der Dichtungshalterung 50 befestigt. Am proximalen Ende weist die Dichtung 42 eine Fassung 46 auf, in die die Abschlüsse 34 der Lamellen 22 formschlüssig angeordnet sind. Weiterhin weist die Dichtung 42 am proximalen Ende eine Öffnung 44 und zwischen der Öffnung 44 und der sie umgebenden Fassung 46 eine Dichtungslippe 45 auf.

Das proximale Ende der Kugelfassung 54 ist mit dem Ringkörper 24 des Spreizkonus 20 verbunden.

Wird z.B. durch den schrägen Versatz eines durchschnittsgeringeren Schaftes eine Kraft auf das Dichtungssystem entsprechend der Richtung des Pfeils 63 ausgeübt, so bewegt sich der Spreizkonus 20 in solch einem beweglichen Hüllkörper mit dem Schaft mit (Fig. 10a). Durch die Bewegung des Spreizkonus 20 in Richtung des Pfeils 63 wird auch die direkt mit diesem verbundene Kugelfassung 54 in gleicher Weise bewegt. Dies ist möglich, da diese drehgelenkig auf dem Mittelteil 52 angeordnet ist. Dieses bewegt sich seinerseits auch in gewissem Maße mit, da es drehgelenkig auf der Dichtungshalterung 50 angeordnet ist.

Die Verbindung zwischen den Lamellen 22 und der Fassung 46 sorgt bei einer solchen Bewegung des Spreizkonus 20 für eine entsprechende Verformung der Dichtung 48. Die Folge ist, dass der Schaft trotz des Versatzes eine zentrale Lage in der Öffnung 44 der Dichtung 48 aufweist und kein Verkanten in der Öffnung 44 sowie keine sich daraus ergebende Undichtigkeit auftritt. Durch das Doppelkugelgelenk 41 passt sich das Dichtungssystem somit an jeglichen Versatz eines Schaftes im medizinischen Instrument an.

Gleiches gilt auch für die flexiblen Hüllrohre 26 (Fig. 1 - 5) und 73 (Fig. 9).

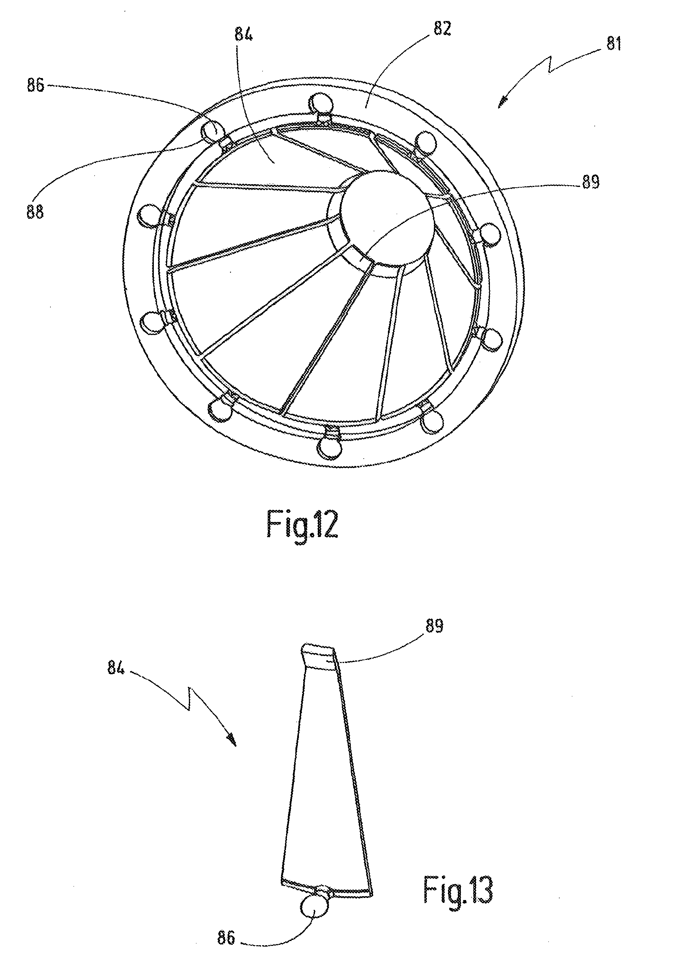

Ein anderes Ausführungsbeispiel für Lamellen ist in Fig. 13 dargestellt. Eine dort dargestellte Lamelle 84 weist am distalen Ende einen Abschluss 89 und am proximalen Ende einen Kugelkopf 86 auf.

Dieser Kugelkopf 86 dient zur beweglichen Verbindung der Lamelle 84 mit einem Ringkörper 82. Hierzu weist der Ringkörper 82 Öffnungen 88 auf, in die die Kugelköpfe 86 eingesetzt werden können. Das Einsetzen der Lamellen ist sowohl als Einrastmechanismus als auch als einfacher Einlegmechanismus denkbar. Für zumindest letztere Variante wäre dann noch ein hier nicht gezeigter Abdeckring nötig, der ein ungewolltes Herabfallen der Lamellen 84 aus dem Ringkörper 82 verhindert (Fig. 12).

Dadurch ergibt sich ein Spreizkonus 81, analog zu dem Spreizkonus 20 mit den Folienscharnieren 34 des zuvor erwähnten Ausführungsbeispiels. Analog zu diesem weisen auch die Lamellen 84 eine Verjüngung von proximal nach distal auf.

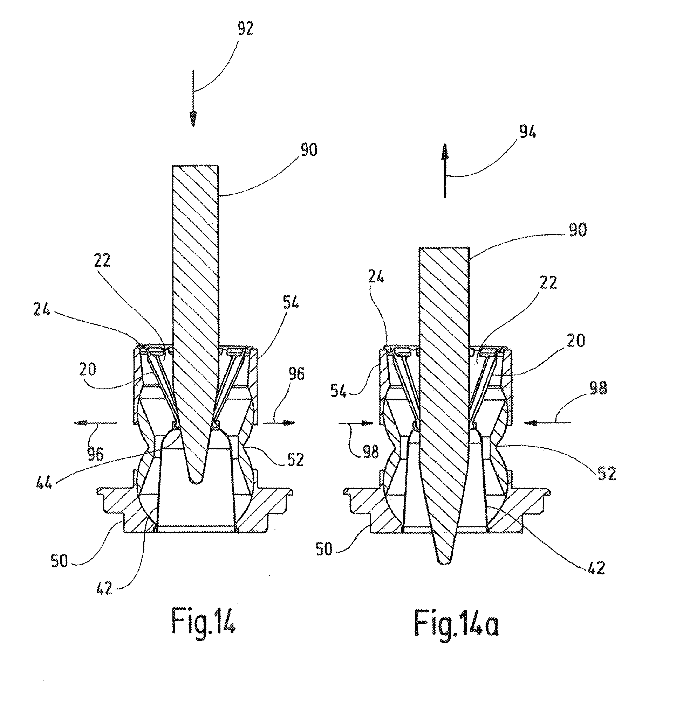

Die Funktionsweise der zuvor beschriebenen Dichtungssysteme ist am Beispiel des Dichtungssystems mit dem Doppelkugelgelenk 41 aus Fig. 10 in den Fig. 14 und 14a beschrieben.

Hierbei wird ein Schaft 90 von proximal her durch den Ringkörper 24 in das Dichtungssystem eingeführt (Fig. 14). Dabei stößt dieser Schaft 90 mit seinem distalen Ende gegen die Lamellen 22 des Spreizkonus 20. Von diesen wird er bei fortlaufender Bewegung in Richtung des Pfeils 92 in Richtung der Öffnung 44 geleitet.

Aufgrund des spitz zulaufenden distalen Endes des Schaftes 90 vergrößert sich dessen Durchmesser auf Höhe der Öffnung 44 bei fortschreitender Bewegung in Richtung des Pfeils 92. Dies hat zur Folge, dass die Abschlüsse 34 der Lamellen 22 weiter nach außen gedrückt werden. Dies ist durch die Pfeile 96 angedeutet. Aufgrund der formschlüssigen Verbindung zwischen den Abschlüssen 34 und der Fassung 46 wird damit auch die Dichtung 42 an ihrer Öffnung 44 in Richtung der Pfeile 96 aufgeweitet. Die Aufweitung der Öffnung 44 durch den Schaft 90 wird somit durch die Lamellen 22 vermittelt.

Aufgrund der die Öffnung 44 umgebenden Dichtungslippe 45 und des stetigen dem Spreizvorgang entgegengerichteten Gegendrucks, verursacht durch die Rückstellkraft des elastischen Materials der Dichtung 42, wird dauernd eine Abdichtung des gesamten Systems beim Einführen des Schaftes 90, wie es in Fig. 14 gezeigt ist, gewährleistet.

Der in Fig. 14a gezeigte maximale Aufweitungszustand in diesem Beispiel wird durch Bewegung des Schaftes 90 in Richtung des Pfeils 94 rückgängig gemacht.

Aufgrund der Rückstellkraft des elastischen Materials der Dichtung 42 übt diese, wie zuvor bereits erwähnt, an der Öffnung 44 einen konstanten Druck auf den Schaft 90 aus. Verringert sich dessen Durchmesser auf Höhe der Öffnung 44 bei der Bewegung in proximale Richtung, so verringert sich auch der Durchmesser der Öffnung 44. Dies ist durch die Pfeile 98 angedeutet. Weiterhin werden auch die Lamellen 22 durch ihre Verbindung zur Dichtung 42 über die Fassung 46 gegen den Schaft 90 gedrückt. Somit gelangen die Lamellen 22 sowie die Dichtung 42 wieder in ihre Ausgangsposition. wie sie in Fig. 10 zu erkennen ist.

## Patentansprüche

1. Medizinisches Instrument, mit einer Kanüle (12), durch die Schäfte (90) weiterer Instrumente mit unterschiedlichen Schaftdurchmessern hindurchführbar sind, mit einer Dichtung (14, 42, 66), die eine Öffnung (16, 44, 70) mit veränderbarem Öffnungsquerschnitt aufweist, durch den die Schäfte (90) mit unterschiedlichen Schaftdurchmesser dicht hindurchführbar sind, mit einer Spreizvorrichtung zum Spreizen der Öffnung (16, 44, 70) der Dichtung (14, 42, 66), wobei die Spreizvorrichtung einen Spreizkonus (20) aufweist, der mehrere Lamellen (22) aufweist, die schwenkbar an einem Ringkörper (24) angebracht sind und einen sich von proximal nach distal verjüngenden Spreizkörper bilden, der mit der Dichtung (14, 42, 66) im Bereich der Öffnung (16, 44, 70) in Verbindung steht, **dadurch gekennzeichnet, dass** die Lamellen (22) über Filmscharniere (34) mit dem Ringkörper (24) verbunden sind.

2. Medizinisches Instrument, mit einer Kanüle (12) durch die Schäfte (90) weiterer Instrumente mit unterschiedlichen Schaftdurchmessern hindurchführbar sind, mit einer Dichtung (14, 42, 66), die eine Öffnung (16, 44, 70) mit veränderbarem Öffnungsquerschnitt aufweist, durch den die Schäfte (90) mit unterschiedlichen Schaftdurchmesser dicht hindurchführbar sind, mit einer Spreizvorrichtung zum Spreizen der Öffnung (16, 44, 70) der Dichtung (14, 42, 66), wobei die Spreizvorrichtung einen Spreizkonus (81) aufweist, der mehrere Lamellen (84) aufweist, die schwenkbar an einem Ringkörper (82) angebracht sind und einen sich von proximal nach distal verjüngenden Spreizkörper bilden, der mit der Dichtung (14, 42, 66) im Bereich der Öffnung (16, 44, 70) in Verbindung steht, **dadurch gekennzeichnet, dass** die Lamellen (84) Kugelköpfe (86) aufweisen über die diese in entsprechende Öffnungen (88) im Ringkörper (82) einhängbar sind.

3. Medizinisches Instrument nach Anspruch 2, **dadurch gekennzeichnet, dass** die Kugelköpfe (86) der Lamellen (84) durch einen Abdeckring im Ringkörper (82) gehalten werden.

4. Medizinisches Instrument nach einem der Ansprüche 1 bis 3, **dadurch gekennzeichnet, dass** sich die Dichtung (14, 42, 66) vom distalen Ende des Spreizkonus (20, 81) nach distal weg erstreckt.

5. Medizinisches Instrument nach einem der Ansprüche 1 bis 3, **dadurch gekennzeichnet, dass** die Dichtung (14, 42, 66) den Spreizkonus (20, 81) umgibt.

6. Medizinisches Instrument nach einem der Ansprüche 1 bis 5, **dadurch gekennzeichnet, dass** das distale Ende der Lamellen (22, 84) des Spreizkonus (20, 81) formschlüssig mit einer Fassung (30, 46, 72) am Rand der Öffnung (16, 44, 70) in der Dichtung (14, 42, 66) verbunden ist.

7. Medizinisches Instrument nach einem der Ansprüche 1 bis 6, **dadurch gekennzeichnet, dass** das distale Ende der Lamellen (22, 84) des Spreizkonus (20, 81) mit der Dichtung (14, 42, 66) verklebt ist.

8. Medizinisches Instrument nach einem der Ansprüche 1 bis 7, **dadurch gekennzeichnet, dass** die Dichtung (14, 42, 66) zusammen mit dem Spreizkonus (20, 81) in einem Hüllkörper angeordnet ist.

9. Medizinisches Instrument nach Anspruch 8, **dadurch gekennzeichnet, dass** der Hüllkörper aus einem Doppelkugelgelenk besteht.

10. Medizinisches Instrument nach Anspruch 9, **dadurch gekennzeichnet, dass** das Doppelkugelgelenk aus einer Dichtungshalterung (50), einem Kugelformen aufweisenden Mittelteil (52) und einer proximalen Kugelfassung (54) besteht.

11. Medizinisches Instrument nach Anspruch 8, **dadurch gekennzeichnet, dass** der Hüllkörper aus einem flexiblen Hüllrohr (26, 73) besteht.

12. Medizinisches Instrument nach Anspruch 11, **dadurch gekennzeichnet, dass** das flexible Hüllrohr (26, 73) aus einer im Wesentlichen parallelen Aneinanderreihung von Ringelementen (38, 74) besteht.

13. Medizinisches Instrument nach Anspruch 12, **dadurch gekennzeichnet, dass** zwei benachbarte Ringelemente (38, 74) an zwei sich radial gegenüberliegenden Stellen miteinander verbunden sind.

14. Medizinisches Instrument nach Anspruch 13, **dadurch gekennzeichnet, dass** die Verbindungsstellen eines Ringelements (38, 74) zu dem distal nächsten Ringelement (38, 74) jeweils immer um 90° gegenüber den Verbindungsstellen zu dem proximal nächsten Ringelement (38, 74) kreisförmig versetzt angeordnet sind.

15. Medizinisches Instrument nach Anspruch 13 oder 14, **dadurch gekennzeichnet, dass** die Verbindungen flexible Stege (40) sind.

16. Medizinisches Instrument nach Anspruch 13 oder 14, **dadurch gekennzeichnet, dass** die Verbindungen Kippgelenke (76) sind.

17. Medizinisches Instrument nach Anspruch 16, **dadurch gekennzeichnet, dass** die Ringelemente (74) an den Stellen einer Kippgelenkfassung (80) höher sind als an denen eines Kippgelenkeinsatzes (78).

18. Medizinisches Instrument nach einem der Ansprüche 11 bis 17, **dadurch gekennzeichnet, dass** sich das flexible Hüllrohr (26, 73) von proximal nach distal konisch aufweitet.

19. Medizinisches Instrument nach einem der Ansprüche 1 bis 18, **dadurch gekennzeichnet, dass** der Spreizkonus (20, 81) aus acht bis zwölf Lamellen (22, 84) besteht.

20. Medizinisches Instrument nach einem der Ansprüche 1 bis 18, **dadurch gekennzeichnet, dass** der Spreizkonus (20, 81) aus zehn Lamellen (22, 84) besteht.

21. Medizinisches Instrument nach einem der Ansprüche 1 bis 20, **dadurch gekennzeichnet, dass** die einzelnen Lamellen (22, 84) von proximal nach distal verjüngen.

22. Medizinisches Instrument nach einem der Ansprüche 1 bis 21, **dadurch gekennzeichnet, dass** die Lamellen (22, 84) aus Hartplastik gefertigt sind.

23. Medizinisches Instrument nach einem der Ansprüche 1 bis 22, **dadurch gekennzeichnet, dass** der Ringkörper (24, 82) aus Hartplastik gefertigt ist.

24. Medizinisches Instrument nach einem der Ansprüche 8 bis 23, **dadurch gekennzeichnet, dass** der Hüllkörper aus Hartplastik gefertigt ist.

25. Medizinisches Instrument nach einem der Ansprüche 1 bis 24, **dadurch gekennzeichnet, dass** die Dichtung (14, 42, 66) aus einem elastischen Material gefertigt ist.

## Claims

1. Medical instrument, with a cannula (12) through which shafts (90) of other instruments having different shaft diameters can be guided, with a seal (14, 42, 66) comprising an opening (16, 44, 70) with a variable opening cross section through which the shafts (90) having different shaft diameters can be guided sealingly, with an expansion device for expanding the opening (16, 44, 70) of the seal (14, 42, 66), which expansion device has an expansion cone (20) with a plurality of slats (22) that are mounted pivotably on an annular body (24) and form an expansion body that narrows from the proximal end to the distal end and that is connected to the seal (14, 42, 66) in the area of the opening (16, 44, 70), **characterized in that** the slats (22) are connected to the annular body (24) via film hinges (34).

2. Medical instrument, with a cannula (12) through which shafts (90) of other instruments having different shaft diameters can be guided, with a seal (14, 42, 66) comprising an opening (16, 44, 70) with a variable opening cross section through which the shafts (90) having different shaft diameters can be guided sealingly, with an expansion device for expanding the opening (16, 44, 70) of the seal (14, 42, 66), which expansion device has an expansion cone (81) with a plurality of slats (84) that are mounted pivotably on an annular body (82) and form an expansion body that narrows from the proximal end to the distal end and that is connected to the seal (14, 42, 66) in the area of the opening (16, 44, 70), **characterized in that** the slats (84) have spherical heads (86) via which they can be engaged in corresponding openings (88) in the annular body (82).

3. Medical instrument according to Claim 2, **characterized in that** the spherical heads (86) of the slats (84) are held in the annular body (82) by a cover ring.

4. Medical instrument according to one of Claims 1 to 3, **characterized in that** the seal (14, 42, 66) extends in the distal direction away from the distal end of the expansion cone (20, 81).

5. Medical instrument according to one of Claims 1 to 3, **characterized in that** the seal (14, 42, 66) surrounds the expansion cone (20, 81).

6. Medical instrument according to one of Claims 1 to 5, **characterized in that** the distal end of the slats (22, 84) of the expansion cone (20, 81) is connected with a form fit to a socket (30, 46, 72) at the edge of the opening (16, 44, 70) in the seal (14, 42, 66).

7. Medical instrument according to one of Claims 1 to 6, **characterized in that** the distal end of the slats (22, 84) of the expansion cone (20, 81) is adhesively bonded to the seal (14, 42, 66).

8. Medical instrument according to one of Claims 1 to 7, **characterized in that** the seal (14, 42, 66) is arranged together with the expansion cone (20, 81) in a casing structure.

9. Medical instrument according to Claim 8, **characterized in that** the casing structure is composed of a double ball joint.

10. Medical instrument according to Claim 9, **characterized in that** the double ball joint is composed of a seal holder (50), a middle part (52) with ball shapes, and a proximal ball socket (54).

11. Medical instrument according to Claim 8, **characterized in that** the casing structure is composed of a flexible casing tube (26, 73).

12. Medical instrument according to Claim 11, **characterized in that** the flexible casing tube (26, 73) is composed of a substantially parallel series of ring elements (38, 74).

13. Medical instrument according to Claim 12, **characterized in that** two adjacent ring elements (38, 74) are connected to each other at two radially opposite sites.

14. Medical instrument according to Claim 13, **characterized in that** the sites connecting a ring element (38, 74) to the distally adjacent ring element (38, 74) are in each case always offset by 90° with respect to the connecting sites to the proximally adjacent ring element (38, 74).

15. Medical instrument according to Claim 13 or 14, **characterized in that** the connections are flexible webs (40).

16. Medical instrument according to Claim 13 or 14, **characterized in that** the connections are tilting joints (76).

17. Medical instrument according to Claim 16, **characterized in that** the ring elements (74) are higher at the sites of a tilting-joint socket (80) than at those of a tilting-joint insert (78).

18. Medical instrument according to one of Claims 11 to 17, **characterized in that** the flexible casing tube (26, 73) widens conically from the proximal end to the distal end.

19. Medical instrument according to one of Claims 1 to 18, **characterized in that** the expansion cone (20, 81) is composed of eight to twelve slats (22, 84).

20. Medical instrument according to one of Claims 1 to 18, **characterized in that** the expansion cone (20, 81) is composed of ten slats (22, 84).

21. Medical instrument according to one of Claims 1 to 20, **characterized in that** the individual slats (22, 84) narrow from the proximal end to the distal end.

22. Medical instrument according to one of Claims 1 to 21, **characterized in that** the slats (22, 84) are made of hard plastic.

23. Medical instrument according to one of Claims 1 to 22, **characterized in that** the annular body (24, 82) is made of hard plastic.

24. Medical instrument according to one of Claims 8 to 23, **characterized in that** the casing structure is made of hard plastic.

25. Medical instrument according to one of Claims 1 to 24, **characterized in that** the seal (14, 42, 66) is made of an elastic material.

## Revendications

1. Instrument médical, avec une canule (12) à travers laquelle des tiges (90) d'autres instruments de différents diamètres de tige peuvent être introduites, avec un joint (14, 42, 66) qui présente une ouverture (16, 44, 70) à section d'ouverture variable à travers laquelle les tiges (90) de différents diamètres de tige peuvent être introduites de manière étanche, avec un dispositif d'écartement pour écarter l'ouverture (16, 44, 70) du joint (14, 42, 66), le dispositif d'écartement présentant un cône d'écartement (20) qui présente plusieurs lamelles (22) qui sont montées pivotantes sur un corps annulaire (24) et forment un corps d'écartement qui se rétrécit du côté proximal vers le côté distal et qui est en liaison avec le joint (14, 42, 66) dans la région de l'ouverture (16, 44, 70), **caractérisé en ce que** les lamelles (22) sont reliées au corps annulaire (24) par l'intermédiaire de charnières film (34).

2. Instrument médical, avec une canule (12) à travers laquelle des tiges (90) d'autres instruments de différents diamètres de tige peuvent être introduites, avec un joint (14, 42, 66) qui présente une ouverture (16, 44, 70) à section d'ouverture variable à travers laquelle les tiges (90) de différents diamètres de tige peuvent être introduites de manière étanche, avec un dispositif d'écartement pour écarter l'ouverture (16, 44, 70) du joint (14, 42, 66), le dispositif d'écartement présentant un cône d'écartement (81) qui présente plusieurs lamelles (84) qui sont montées pivotantes sur un corps annulaire (82) et forment un corps d'écartement qui se rétrécit du côté proximal vers le côté distal et qui est en liaison avec le joint (14, 42, 66) dans la région de l'ouverture (16, 44, 70), **caractérisé en ce que** les lamelles (84) présentent des têtes sphériques (86) par lesquelles celles-ci peuvent être accrochées dans des ouvertures (88) correspondantes dans le corps annulaire (82).

3. Instrument médical selon la revendication 2, **caractérisé en ce que** les têtes sphériques (86) des lamelles (84) sont maintenues dans le corps annulaire (82) par un anneau de recouvrement.

4. Instrument médical selon l'une des revendications 1 à 3, **caractérisé en ce que** le joint (14, 42, 66) s'étend en s'éloignant distalement de l'extrémité distale du cône d'écartement (20, 81).

5. Instrument médical selon l'une des revendications 1 à 3, **caractérisé en ce que** le joint (14, 42, 66) entoure le cône d'écartement (20, 81).

6. Instrument médical selon l'une des revendications 1 à 5, **caractérisé en ce que** l'extrémité distale des lamelles (22, 84) du cône d'écartement (20, 81) est relié par complémentarité de forme à un logement (30, 46, 72) au bord de l'ouverture (16, 44, 70) dans le joint (14, 42, 66).

7. Instrument médical selon l'une des revendications 1 à 6, **caractérisé en ce que** l'extrémité distale des lamelles (22, 84) du cône d'écartement (20, 81) est collée avec le joint (14, 42, 66).

8. Instrument médical selon l'une des revendications 1 à 7, **caractérisé en ce que** le joint (14, 42, 66) est disposé conjointement avec le cône d'écartement (20, 81) dans un corps enveloppant.

9. Instrument médical selon la revendication 8, **caractérisé en ce que** le corps enveloppant est constitué d'une double articulation sphérique.

10. Instrument médical selon la revendication 9, **caractérisé en ce que** la double articulation sphérique est constituée d'un support de joint (50), d'une partie centrale (52) présentant des formes sphériques et d'un logement sphérique proximal (54).

11. Instrument médical selon la revendication 8, **caractérisé en ce que** le corps enveloppant est constitué d'un tube enveloppant souple (26, 73).

12. Instrument médical selon la revendication 11, **caractérisé en ce que** le tube enveloppant souple (26, 73) est constitué d'une succession sensiblement parallèle d'éléments annulaires (38, 74).

13. Instrument médical selon la revendication 12, **caractérisé en ce que** deux éléments annulaires (38, 74) adjacents sont reliés entre eux à deux endroits radialement opposés.

14. Instrument médical selon la revendication 13, **caractérisé en ce que** les points de liaison d'un élément annulaire (38, 74) avec l'élément annulaire (38, 74) le plus proche côté distal sont toujours décalés circulairement de 90° par rapport aux points de liaison avec l'élément annulaire (38, 74) le plus proche côté proximal.

15. Instrument médical selon la revendication 13 ou 14, **caractérisé en ce que** les liaisons sont des entretoises souples (40).

16. Instrument médical selon la revendication 13 ou 14, **caractérisé en ce que** les liaisons sont des articulations de basculement (76).

17. Instrument médical selon la revendication 16, **caractérisé en ce que** les éléments annulaires (74) sont plus élevés aux endroits d'un logement d'articulation de basculement (80) qu'à ceux d'un insert d'articulation de basculement (78).

18. Instrument médical selon l'une des revendications 11 à 17, **caractérisé en ce que** le tube enveloppant souple (26, 73) s'élargit en cône du côté proximal vers le côté distal.

19. Instrument médical selon l'une des revendications 1 à 18, **caractérisé en ce que** le cône d'écartement (20, 81) est composé de huit à douze lamelles (22, 84).

20. Instrument médical selon l'une des revendications 1 à 18, **caractérisé en ce que** le cône d'écartement (20, 81) est composé de dix lamelles (22, 84).

21. Instrument médical selon l'une des revendications 1 à 20, **caractérisé en ce que** les différentes lamelles (22, 84) se rétrécissent du côté proximal vers le côté distal.

22. Instrument médical selon l'une des revendications 1 à 21, **caractérisé en ce que** les lamelles (22, 84) sont réalisées en plastique dur.

23. Instrument médical selon l'une des revendications 1 à 22, **caractérisé en ce que** le corps annulaire (24, 82) est réalisé en plastique dur.

24. Instrument médical selon l'une des revendications 8 à 23, **caractérisé en ce que** le corps enveloppant est réalisé en plastique dur.

25. Instrument médical selon l'une des revendications 1 à 24, **caractérisé en ce que** le joint (14, 42, 66) est réalisé dans une matière élastique.
